Europäisches Patentamt

European Patent Office

Office européen des brevets

(11) Publication number: **0 407 965 A1**

## EUROPEAN PATENT APPLICATION

(21) Application number: **90113152.4**

(22) Date of filing: **10.07.90**

(51) Int. Cl.5: **A61M 25/00, A61M 25/01**

(30) Priority: **10.07.89 JP 177684/89**

(43) Date of publication of application:
**16.01.91 Bulletin 91/03**

(84) Designated Contracting States:
**BE DE FR GB IT NL SE**

(71) Applicant: **Terumo Kabushiki Kaisha**
**No. 44-1, Hatagaya 2-chome Shibuya-ku**
**Tokyo 151(JP)**

(72) Inventor: **Miyano, Yasuo**
**c/o Terumo Kabushiki Kaisha, 150,**
**Maimaigi-cho**
**Fujinomiya-shi, Shizuoka(JP)**

(74) Representative: **Casalonga, Axel et al**
**BUREAU D.A. CASALONGA - JOSSE**
**Morassistrasse 8**
**D-8000 München 5(DE)**

(54) **Guide wire.**

(57) The guide wire (1) according to the present invention comprises a core (2); and a synthetic resin layer (3) for covering the outer surface of the core (2) and containing fluororesin. Therefore, an excellent slidability in the blood vessel and the catheter can be obtained and a proper sliding performance can be obtained with which the guide wire can be assuredly held with the hand with a vital adaptability permanently exhibited. Furthermore, the guide wire (1) according to the present invention comprises: a core (2); a layer (4) for covering the outer surface of the core (2) and having contrast characteristics; and a synthetic resin layer (3) for covering the outer surface of the layer (4) having the contrast characteristics and containing fluororesin. Therefore, the position of the overall body of the guide wire can be easily confirmed under the X-ray contrast. In particular, the position of the front portion of the guide wire can be easily confirmed. Therefore, the handling facility can be improved. Since the core (2) is made of ultraelastic metal, any viciousness due to the bending operation can be prevented and its flexibility can be maintained. Therefore, the guide wire (1) can be easily inserted into the blood vessel with a good correspondence maintained even the blood vessel meanders.

FIG.1

FIG.2

EP 0 407 965 A1

## GUIDE WIRE

The present invention relates to a guide wire which is inserted into a blood vessel prior to the insertion of a catheter for performing the inspection and treatment of the inside portion of the blood vessel by using a catheter.

Recently, inspections and treatments of cardiopathy by introducing a catheter into a blood vessel have been performed. When the catheter is introduced into a desired position of the patient's body, a guide wire is inserted into the catheter in such a manner that the front portion of the guide wire projects slightly forward of the front portion of the catheter so that the catheter is guided to the desired position.

In order to move the guide wire to the desired position in the blood vessel, an excessively complicated and skilled operation, in which the guide wire is pushed, pulled and rotated, is necessary. Therefore, too long a time is required to complete the operation. In particular, since the arteries of the aged meander to an extreme degree due to arteriosclerosis or the like, more time is required to insert the guide wire than for young people. Therefore, an attempt has been made in that the guide wire is coated with a material exhibiting an excellent vital adaptability. Materials exhibiting an excellent vital adaptability are exemplified by fluororesin and silicone. Furthermore, polyurethane resin has been disclosed in Japanese Patent Laid-Open No. 60-12069. However, the above-described spring type guide wire coated with the above-described fluororesin arises a problem in that the coating material may crack or become separated when the guide wire passes through the meandering blood vessel.

Another attempt has been made in that the guide wire is coated with fluororesin in order to smooth the surface of the guide wire for the purpose of shortening the time taken for the guide wire to reach its desired position. However, the above-described structure raises a problem in that the coating material may crack and become separated when the guide wire passes through the meandering blood vessel. The above-described separation or the like must be avoided since the cracks cause a thrombus to be generated.

Furthermore, in order to improve sliding facility, a guide wire, arranged in such a manner that a water soluble high polymer is bonded to its surface, has been disclosed in Japanese Patent Laid-Open No. 61-45775. A guide wire of this type can easily be inserted since the resistance taken place with respect to the catheter can be reduced because the guide wire can easily be slid due to the water soluble high polymer. However, since the guide wire is directly held with the hand, the operations of pushing/pulling/rotating the guide wire cannot be easily performed due to the slipping. In particular, when the guide wire is handled so as to retain the catheter in the coronary arteries, the property of easy manual handling has been considered more important than the sliding facility.

An object of the present invention is to overcome the above-described problems experienced with the conventional structure. Therefore, an object of the present invention is to provide a guide wire exhibiting an excellent vital adaptability and also having a smooth and properly slidable surface (that is, a guide wire capable of sliding in the blood vessel and the catheter and able to be held confidently with the hand).

The above-described object can be achieved by a guide wire comprising: a core; and a synthetic resin layer for covering the outer surface of the core and containing fluororesin.

The above-described object can be achieved by a guide wire comprising: a core; a layer for covering the outer surface of the core and having contrast characteristics; and a synthetic resin layer for covering the outer surface of the layer having the contrast characteristics and containing fluororesin.

It is preferable that the above-described proportion of the fluororesin in the synthetic resin layer is 20 to 60 wt%.

It is preferable that the above-described core is made of super-elastic metal.

## BRIEF DESCRIPTION OF DRAWINGS

Fig. 1 is a cross sectional view which illustrates an embodiment of a guide wire according to the present invention;

Fig. 2 is a cross sectional view which illustrates another embodiment of the guide wire according to the present invention;

Fig. 3 illustrates the way of carrying out Experiment 1;

Fig. 4 illustrates the way of carrying out Experiment 2; and

Fig. 5 illustrates the way of carrying out Experiment 3.

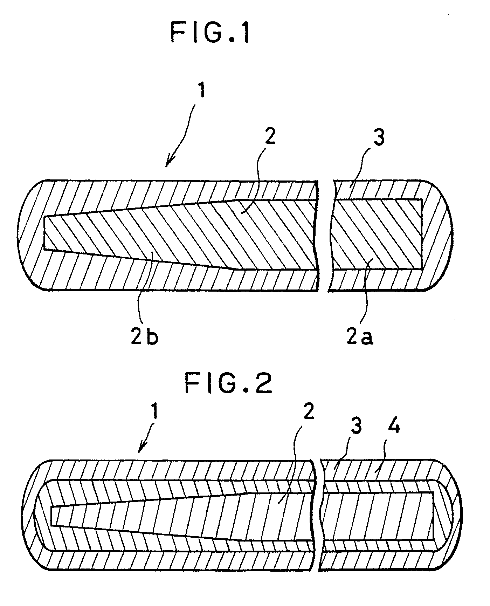

An embodiment of a guide wire according to the present invention will be described with reference to the drawings. Fig. 1 is a cross sectional view which illustrates an embodiment of a guide wire according to the present invention. A guide wire 1 according to the present invention comprises a core 2; and a synthetic resin layer 3 for covering the outer surface of the core 2 and containing fluororesin. Then, the guide wire according to the present invention will be described with reference to Fig. 1.

The core 2 of the guide wire 1 comprise a body 2a and a front portion 2b which are integrally formed by super-elastic metal. The front portion 2b is arranged in such a manner that its diameter is gradually reduced toward its front terminal from the front terminal of the body 2a. As a result of the structure arranged in such a manner that the diameter is gradually reduced, the front portion can be gradually warped when force acts on the front terminal. Therefore, the flexibility is increased toward the front terminal. As a result, the inner wall of the blood vessel can be protected from damage. Also the front portion can be softened by subjecting the front portion and the other portions to different heat treatment conditions at the time of manufacturing the core or after the manufacturing.

As the core 2, an super-elastic metal such as an Ni-Ti alloy, Cu-Zn alloy and the like is employed. For example, an super-elastic metal such as an Ni-Ti alloy composed by 48 to 58 atom % of Ni (the balance: Ti), an Ni-Ti-X alloy (X = Fe, Si, Al, V), a Cu-Zn alloy composed by 38.5 to 41.5 wt% of Zn (the balance: Cu), a Cu-Zn-X alloy (X = Be, Si, Sn, Al, Ga) composed by 1 to 10 wt% of X and 38.5 to 41.5 wt% of Zn (the balance: Cu), an Ni-Al alloy composed by 36 to 38 wt% of Al is preferably used. The core 2 may be formed by a sole or a plurality of metal wires such as stainless steel wires and piano wires arranged in the axial direction in a straight form or twined manner.

The outer diameter of the body 2a of the core is 0.10 to 1.00 mm, preferably 0.50 to 0.80 mm. The length is 1000 to 4000 mm, preferably 1500 to 3000 mm. The buckling strength (yield stress when loaded) is 30 to 100 $kgf/mm^2$ (22 °C), preferably 40 to 60 $kgf/mm^2$. The reversion stress (yield stress when load has been removed) is 20 to 30 $kgf/mm^2$ (22 °C), preferably 30 to 40 $kgf/mm^2$.

The outer diameter of the front portion 2b of the core 2 is 0.03 to 0.20 mm, preferably 0.05 to 0.15 mm. The length is 5 to 300 mm, preferably 20 to 150 mm. Thebending load is 1.0 to 10 gf, preferably 1.5 to 6.0 gf. The reversion stress is 1.0 to 10 gf, preferably 1.5 to 6.0 gf.

The structure may be formed in such a manner that the shape can be optionally changed by subjecting a portion of predetermined length from the front terminal of the front portion 2b to a heat treatment.

Furthermore, a member (for example, gold, platinum, lead, a bismuth oxide) having high X-ray contract characteristics may be fixed to the front terminal of the front portion 2b. As a result of this structure, the position of the front portion of the guide wire can be clearly identified.

The synthetic resin layer 3 covering the overall body of the core 2 has, as shown in Fig. 1, a substantially equal outer diameter including its front portion. The synthetic resin layer 3 comprises a base made of polyethylene, polyvinyl chloride, polyester, polypropylene, polyamide, polyurethane, polystyrene, silicone rubber, or an elastomer of each of the above-described materials and a material composed by combining the above-described materials, the base containing fluororesin.

As the fluororesin, polytetrafluoroethylene (PTFE), polychlorotrifluoroethylene (PCTFE), tetrafluoroethylenehexafluoroprophylene copolymer (FEP), tetrafluoroethyleneethylene copolymer (ETFE) and the like is preferably used.

The proportion of the fluororesin in the synthetic resin layer 3 is 20 to 60 wt%, preferably 30 to 50 wt%. If the content is less than 20 wt%, the vital adaptability and the sliding facility due to the fluororesin cannot be satisfactorily exhibited. If the content exceeds 60 wt%, the surface smoothness of the synthetic resin layer 3 is lost, causing proper sliding facility to be lost.

As for the method of manufacturing the guide wire 1 according to the embodiment shown in Fig. 1, it can be manufactured by, for example, extruding the solved synthetic resin containing fluororesin together with the material for the core so as to have the core coated with the synthetic resin.

Then, another embodiment shown in Fig. 2 will be described. The guide wire according to this embodiment comprises: a core 2; a layer 4 for covering the outer surface of the core and having contrast characteristics; and a synthetic resin layer 3 for covering the outer surface of the layer having the contrast characteristics and containing fluororesin.

The layer 4, which acts as the characteristic portion of the guide wire according to this embodiment, which is disposed between the core 2 and the synthetic resin layer 3 and which has the contrast characteristics, comprises a base made of polyethylene, polyvinyl chloride, polyester, polypropylene, polyamide, polyurethane, polystyrene, silicone rubber, or the elastomer of each of the above-described materials and a material composed by combining the above-described materials, the base being mixed with pulverized X-ray contrast material such as sole or a compound of Ba, W, Bi, Pb, Ti or the like. As a result, the position of the overall body of the guide wire 1 introduced into the blood vessel can be easily confirmed. IT is preferable that the proportion of the X-ray contrast material be 20 to 70 wt%, further

3

preferably 35 to 55 wt%. If the content is less than 20 wt%, the position confirmation of the guide wire introduced into the blood vessel cannot be easily conducted. If it exceeds 70 wt%, the physical properties of the plastic in the synthetic resin layer 4 deteriorate, causing its molding to become difficult (or become impossible).

The sliding facility of the guide wire according to the above-described two embodiments can be adjusted by apllying a silicone coat to the outer surface of the guide wire.

(Operation)

Then, the operation of the guide wire according to the present invention will be described with reference to Embodiment 1 shown in Fig. 1.

In a state where the front portion of the guide wire 1 is allowed to project over the front portion of a catheter (omitted from illustration), the guide wire 1 is, together with a catheter, introduced into the blood vessel. As a result of the action of guiding the front portion of the catheter performed by the front portion of the guide wire 1, the catheter is inserted into a predetermined position in the blood vessel. During this operation, the guide wire 1 and the catheter are moved forward with the front portions of the guide wire 1 and the catheter confirmed from outside by means of the X-ray contrast manner. After the front portion of the catheter has reached the position adjacent to the desired position, the guide wire is removed from the catheter.

(Embodiments)

The guide wire structured as shown in Fig. 1 is arranged to be Embodiment 1. The Embodiment 1 is structured as follows:

≪Embodiment 1≫

Synthetic resin layer: Polyester elastomer (manufactured by Toray Dupont, trade name: Hytrel 4767) + 30 wt% of PTFE powder (manufactured by Daikin Kogyo, trade name: Lubron)
Core: Ni-Ti alloy (52 atom of Ni, the balance Ti; manufactured by Tokin)
Length of the front portion of the core: 100 mm Thickness of the synthetic resin layer in the body: 0.15 mm
Outer diameter of the guide wire : 0.89 mm (0.035″)

≪Embodiment 2≫

A guide wire formed by applying a silicone treatment (the guide wire is dipped in silicone/Freon solution for 10 seconds before being dried; density of the solution: 5 wt%, silicone: manufactured by Toshiba Silicone, trade name: NCT-911) to the surface of the guide wire according to Embodiment 1 is arranged to Embodiment 2.

Then, the guide wire shown in Fig. 2 is arranged to be Embodiment 3. Its details are as follows:

≪Embodiment 3≫

Synthetic resin layer: Same as Embodiment 1
Layer having contrast characteristics: Polyester elastomer (manufactured by Toray Dupont, trade name: Hytrel 4057) + 25 wt% of $Bi_2O_3$ (manufacturedby Nihon Kagaku Sangyo, trade name: Filler FP)
Core: Same as Embodiment 1
Length of the front portion of the core: 100 mm Thickness of synthetic resin layer of the body: 0.15 mm
Thickness of the layer having the contrast characteristics in the body: 0.12 mm
Outer diameter of the guide wire: Same as Embodiment 1
Silicone treatment: Same as Embodiment 2

Then, guide wires having the same outer diameter (0.89 mm (0.035")) were arranged to be comparative examples as follows:

≪Comparative Example 1≫

(manufactured by Terumo Corporation, trade name : Radifocus ®. Guide wire M)
Structure: Core made of ultraelastic alloy, a synthetic resin layer covering the core, and a water soluble high polymer the surface of which is coated with a synthetic resin layer
Core: Ni-Ti alloy (52 atom % of Ni, the balance: Ti)
Synthetic resin layer: Polyurethane (manufactured by Dainippon Ink, trade name: Pandex)
Water soluble high polymer: Tetrahydrofran + 5.0 wt% maleic anhydride ethylester copolymer

≪Comparative Example 2≫

(Manufactured by Cook Inc., trade name: WIRE GUIDE, product code: TSFNB)
Structure: Spring type (core is disposed in a spring portion) the surface of which is coated with teflon.
Overall length: 1450 mm
Surface treatment: Teflon® baked coating
Material: SUS 304 for both the spring portion and the core.

≪Comparative Example 3≫

(Manufactured by C.R.Bard, Inc., trade name : USCI®,
Teflon® Coating Guide Wire, product code: 007046)
Structure: Spring type (core is disposed in a spring portion) the surface of which is coated with teflon.
Overall length: 1450 mm
Surface treatment: Teflon® baked coating
Material: SUS304 for both the spring portion and the core.

≪Comparative Example 4≫

(Manufactured by Cordis Corporation, trade name : EMERALDTM GUIDEWIRE, product code: S02-521)
Structure: Spring type (core is disposed in a spring portion) the surface of which is coated with teflon.
Overall length: 1500 mm
Surface treatment: Teflon® baked coating
Material: SUS 304 for both the spring portion and the core.
The above-described embodiments and comparative examples were subjected to the following experiments, resulting as shown in Tables 1 and 2.

(Experiment 1: Sliding resistance test)

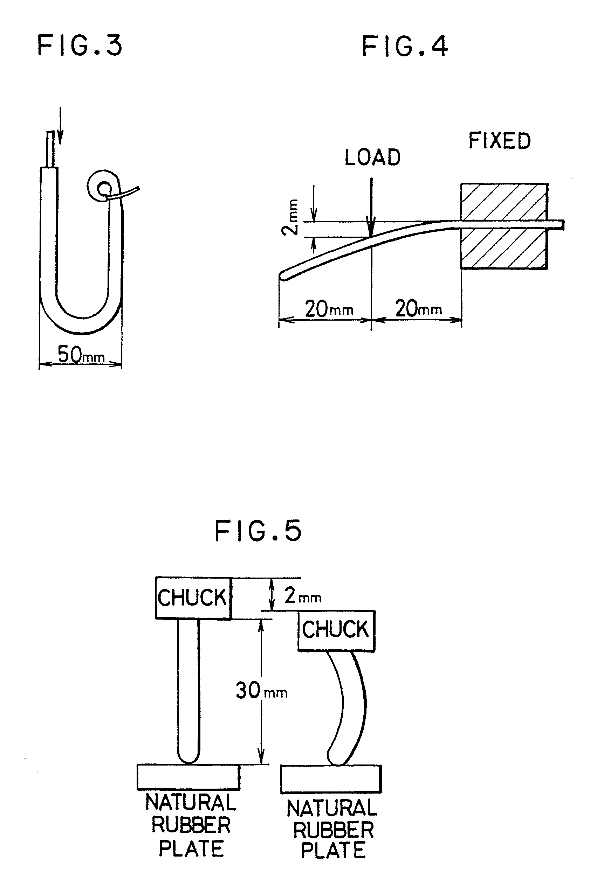

As shown in Fig. 3, a big tail catheter (manufactured by Terumo Corporation, trade name : Radifocus® ) was bent by 180 degrees to have a diameter of 50 mm. The inside portion of the catheter was then filled with water so that the maximum sliding resistance when the guide wire was inserted into the catheter was measured by a compressive tension tester (Shimazu Autograph AGS series AGS-100A)

(Experiment 2: Front portion bending test)

As shown in Fig. 4, the guide wire was fixed at a position from its front terminal by a distance of 40mm so that the load necessary to warp the position away from the front

Table 1

| | Experiment 1 (gf) | Experiment 2 (gf) | Experiment 3 (angle) |
|---|---|---|---|
| Enbodiment 1 | 83 | 1.2 | 0 |
| Enbodiment 2 | 150 | - | 0 |
| Comparative Example 1 | 42 | 0.5 | 0 |
| Comparative Example 2 | 120 | 1.7 | 116 |
| Comparative Example 3 | 131 | 1.4 | 112 |
| Comparative Example 4 | 264 | 1.3 | 122 |

Table 2

| | the shape of the front portion * | Experiment 4 (gf) |
|---|---|---|
| Enbodiment 1 | A | 1.8 |
| Comparative Example 1 | S | 4.7 |
| Comparative Example 1 | A | 0.8 |
| Comparative Example 2 | S | 1240 |
| Comparative Example 3 | S | 1200 |
| Comparative Example 3 | J | 8.7 |
| Comparative Example 4 | S | 20.8 |

\* A~Angle
J~3mmJ
S~straight

terminal by a distance of 20 mm by a degree of the warp of 2 m was measured by the compression tension tester (Shimazu Autograph AGS series AGS-100A)

(Experiment 3: Body Reversion Test)

The body of the guide wire was wound around a circular rod having a diameter of 5 mm so as to make an angle of 180 degrees so that the residual strain angle when the guide wire was removed was measured.

(Experiment 4: Front abutting test)

As shown in Fig. 5, the guide wire was fixed at the position away from its front terminal by a distance of 30 mm so that the load applied when the fixed chuck was lowered by 2 mm with respect to natural rubber was measured by the compression tension tester (Shimazu Autograph AGS series AGS-100A).

As is shown from the thus carried out experiments, it is apparent that the guide wire according to the present invention exhibits proper slidability but has a flexible front portion exhibiting good reversion characteristics.

(Effect of the Invention)

The guide wire according to the present invention comprises a core; and a synthetic resin layer for covering the outer surface of the core and containing fluororesin. Therefore, an excellent slidability in the blood vessel and the catheter can be obtained and a proper sliding performance can be obtained with which the guide wire can be assuredly held with the hand with a vital adaptability permanently exhibited. Furthermore, the guide wire according to the present invention comprises: a core; a layer for covering the outer surface of the core and having contrast characteristics; and a synthetic resin layer for covering the outer surface of the layer having the contrast characteristics and containing fluororesin. Therefore, the position of the overall body of the guide wire can be easily confirmed under the X-ray contrast. In particular, the position of the front portion of the guide wire can be easily confirmed. Therefore, the handling facility can be improved. Since the core is made of ultraelastic metal, any viciousness due to the bending operation can be prevented and its flexibility can be maintained. Therefore, the guide wire can be easily inserted into the blood vessel with a good correspondence maintained even the blood vessel meanders.

## Claims

1. A guide wire comprising: a core; and a synthetic resin layer for covering the outer surface of said core and containing fluororesin.

2. A guide wire comprising: a core; a layer for covering the outer surface of said core and having contrast characteristics; and a synthetic resin layer for covering the outer surface of said layer having the contrast characteristics and containing fluororesin.

3. A guide wire according to claim 1 or 2, wherein said proportion of the fluororesin in the synthetic resin layer is 20 to 60 wt%.

4. A guide wire according to claim 1 or 2, wherein said core is made of super-elastic metal.

# FIG.1

# FIG.2

FIG.3

FIG.4

50mm

LOAD

FIXED

2 mm

20mm  20mm

FIG.5

CHUCK

2 mm

CHUCK

30 mm

NATURAL
RUBBER
PLATE

NATURAL
RUBBER
PLATE

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,P | US-A-4 895 168  (MACHEK)<br>* Figure 1; column 3, line 68 - column 4, line 1; claim 1 *<br>– – – | 1 | A 61 M 25/00<br>A 61 M 25/01 |
| X | EP-A-0 255 234  (MEADOX SURGIMED A/S)<br>* Figures 1-3; column 4, lines 31-36; column 6, lines 35-40 *<br>– – – | 1,2 | |
| X | US-A-4 080 706  (HEILMAN et al.)<br>* Figures 1a,2a,2b; column 4, lines 29-34 *<br>– – – | 1 | |
| Y | RADIOLOGY, vol. 166, no. 2, February 1988, pages 545,546; K. TAKAYASU et al.: "Plastic-coated guide wire for hepatic arteriography"<br>– – – | 1,4 | |
| Y | US-A-4 536 179  (ANDERSON et al.)<br>* Column 1, lines 47-49; column 4, lines 59-67 *<br>– – – | 1,4 | |
| A | | 2 | |
| A | GB-A-2 016 482  (BAXTER TRAVENOL LABORATORIES INC.)<br>* Page 1, lines 48-57,47-99 *<br>– – – – – | 2,3 | |

TECHNICAL FIELDS
SEARCHED (Int. Cl.5)

A 61 M

The present search report has been drawn up for all claims

| Place of search | Date of completion of search | Examiner |
|---|---|---|
| The Hague | 23 October 90 | SEDY, R. |